(19) Europäisches Patentamt
European Patent Office
Office européen des brevets

(11) Veröffentlichungsnummer: **0 007 498**
**A1**

(12) # EUROPÄISCHE PATENTANMELDUNG

(21) Anmeldenummer: 79102313.8

(22) Anmeldetag: 09.07.79

(51) Int. Cl.[3]: C 07 C 125/03
C 07 C 125/00

(30) Priorität: 14.07.78 DE 2830969

(43) Veröffentlichungstag der Anmeldung: 06.02.80 Patentblatt 80/3

(84) Benannte Vertragsstaaten: BE CH DE FR GB IT

(71) Anmelder: BASF Aktiengesellschaft
Carl-Bosch-Strasse 38
D-6700 Ludwigshafen(DE)

(72) Erfinder: Koenig, Karl-Heinz, Dr. Chem.
Pierstrasse 8 a
D-6710 Frankenthal(DE)

(72) Erfinder: Feuerherd, Karl-Heinz, Dr. Chem.
Berner Weg 34
D-6700 Ludwigshafen(DE)

(54) **Neue alpha-Halogenalkylcarbamidsäurehalogenide und Verfahren zur Herstellung von alpha-Halogenalkylcarbamidsäurehalogeniden.**

(57) Neue α-Halogenalkylcarbamidsäurehalogenide und ein Verfahren zur Herstellung von α- Halogenalkylcarbamidsäurehalogeniden durch Umsetzung von Alkenylisocyanaten mit Halogenwasserstoff.

Die nach dem Verfahren der Erfindung hergestellten α-Halogenalkylcarbamidsäurehalogenide sind wertvolle Ausgangsstoffe für die Herstellung von Lackrohstoffen, Textilbeschichtungsmitteln, Farbstoffen, Pharmaceutica und Pflanzenschutzmitteln.

EP 0 007 498 A1

Neue α-Halogenalkylcarbamidsäurehalogenide und Verfahren zur Herstellung von α-Halogenalkylcarbamidsäurehalogeniden

Die Erfindung betrifft neue α-Halogenalkylcarbamidsäurehalogenide und ein Verfahren zur Herstellung von α-Halogenalkylcarbamidsäurehalogeniden durch Umsetzung von Alkenylisocyanaten mit Halogenwasserstoff.

Es ist aus der Angewandten Chemie, Band 74 (1962), Seiten 848 bis 855, bekannt, daß man Alkylcarbamidsäurechloride mit elementarem Chlor in Gemische von Chloralkylcarbamidsäurechloriden und mehrfach chlorierten Produkten umsetzen kann. Die dabei anfallenden Produkte sind jedoch schwer trennbare Gemische sowohl mit Bezug auf den Halogenierungsgrad wie auf die Stellung der eintretenden Halogenatome. Das Verfahren ist im Hinblick auf Ausbeute und Reinheit der Endstoffe sowie einfachen und wirtschaftlichen Betrieb nicht befriedigend, definierte Einzelstoffe, insbesondere Mono-α-halogenalkylcarbamoylchloride, können nicht in wesentlicher Menge isoliert werden.

Es wurde nun gefunden, daß man α-Halogenalkylcarbamidsäurehalogenide der Formel

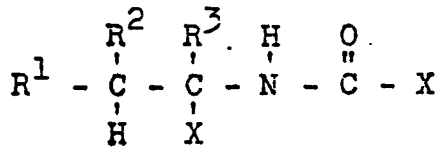 I,

worin die einzelnen Reste $R^1$, $R^2$ und $R^3$ gleich oder verschieden sein können und jeweils einen aliphatischen, cycloaliphatischen, araliphatischen, aliphatisch-aromatischen oder aromatischen Rest bedeuten, $R^1$ und $R^2$ zusammen mit dem benachbarten Kohlenstoffatom oder $R^2$ und $R^3$ zusammen mit den beiden benachbarten Kohlenstoffatomen auch Glieder eines alicyclischen Ringes bezeichnen können, ein oder zwei der Reste $R^1$, $R^2$ und $R^3$ auch jeweils für ein Wasserstoffatom stehen können, X ein Halogenatom bedeutet, vorteilhaft erhält, wenn man Alkenylisocyanate der Formel

$$R^1 - \underset{\substack{| \\ R^2}}{C} = \underset{\substack{| \\ R^3}}{C} - N = C = O \qquad \text{II,}$$

worin $R^1$, $R^2$ und $R^3$ die vorgenannte Bedeutung besitzen, mit Halogenwasserstoff bei einer Temperatur von $-78^\circ C$ bis $+80^\circ C$ umsetzt.

Weiterhin wurden die neuen α-Halogenalkylcarbamidsäurehalogenide der Formel

$$R^1 - \underset{\substack{| \\ H}}{\overset{\substack{R^2 \\ |}}{C}} - \underset{\substack{| \\ X}}{\overset{\substack{R^3 \\ |}}{C}} - N - C - X \qquad \text{I,}$$

worin die einzelnen Reste $R^1$, $R^2$ und $R^3$ gleich oder verschieden sein können und jeweils einen Alkylrest mit 1 bis 12 Kohlenstoffatomen, einen Cycloalkylrest mit 5 bis 6 Kohlenstoffatomen, einen Aralkylrest oder Alkylarylrest mit 7 bis 12 Kohlenstoffatomen, einen Phenylrest bedeuten, $R^1$ und $R^2$ zusammen mit dem benachbarten Kohlenstoffatom oder $R^2$ und $R^3$ zusammen mit den beiden benachbarten Kohlenstoffatomen auch Glieder eines alicyclischen Ringes bezeichnen können, ein oder zwei der Reste $R^1$, $R^2$ und $R^3$ auch jeweils für

ein Wasserstoffatom stehen können, X ein Halogenatom bedeutet, wobei, wenn $R^2$ und $R^3$ gleichzeitig ein Wasserstoffatom bedeuten, $R^1$ für einen cycloaliphatischen, araliphatischen, aliphatisch-aromatischen oder aromatischen Rest steht, gefunden.

Die Umsetzung kann im Falle der Verwendung von Chlorwasserstoff und Buten-1-yl-isocyanat durch die folgenden Formeln wiedergegeben werden:

$$CH_3\text{-}CH_2\text{-}CH{=}\overset{H}{C}\text{-}N{=}CO + 2\ HCl \longrightarrow CH_3\text{-}CH_2\text{-}CH_2\text{-}\underset{Cl}{\overset{H}{C}}\text{-}\overset{H}{N}\text{-}\overset{O}{\overset{\|}{C}}\text{-}Cl\ .$$

Im Hinblick auf das bekannte Verfahren liefert das Verfahren nach der Erfindung auf einfacherem und wirtschaftlicherem Wege α-Halogenalkylcarbamidsäurehalogenide in besserer Ausbeute und Reinheit. Die Aufarbeitung ist wesentlich einfacher, da kein komponentenreiches Reaktionsgemisch entsteht und definierte Endstoffe erhalten werden. Alle diese vorteilhaften Ergebnisse sind überraschend, denn angesichts der sehr reaktionsfreudigen Ausgangsstoffe war mit der Bildung verschiedenartiger Reaktionsprodukte zu rechnen. Auch war zu vermuten, daß α,ß-ungesättigte Stickstoffverbindungen sehr leicht unter der Einwirkung von Säuren polymerisieren oder hydrolysieren. So geht z.B. N-Vinylpyrrolidon durch Einwirkung schon geringer Mengen an anorganischen Säuren in ein Gemisch von Oligomeren über (Ullmanns Encyklopädie der technischen Chemie, Band 14, Seite 261). Bull. Soc. Chim. Belg., Band 65, Seiten 291 bis 296 (1956) zeigt, daß Vinylisocyanat mit wäßriger 12-N-Salzsäure in Aceton zu Acetaldehyd hydrolysiert.

Die Ausgangsstoffe können z.B. durch Umsetzung von entsprechend substituierten Acrylsäurehalogeniden mit Natriumazid (Bull., loc. cit.) oder durch thermische Zerlegung von N-tert.-Butyl-N-alkenylcarbamidsäurehalogeniden hergestellt werden. Man verwendet den Halogenwasserstoff, vorteilhaft Jodwasserstoff, bevorzugt Bromwasserstoff und insbesondere Chlorwasserstoff, in stöchiometrischer Menge oder im Überschuß, vorzugsweise in einer Menge von 2 bis 2,2 Mol Halogenwasserstoff je Mol Ausgangsstoff II.

Bevorzugte Ausgangsstoffe II und dementsprechend bevorzugte Ausgangsstoffe I sind solche, in deren Formeln die einzelnen Reste $R^1$, $R^2$ und $R^3$ gleich oder verschieden sein können und jeweils einen Alkylrest mit 1 bis 12 Kohlenstoffatomen, einen Cycloalkylrest mit 5 bis 6 Kohlenstoffatomen, einen Aralkylrest oder Alkylarylrest mit 7 bis 12 Kohlenstoffatomen, einen Phenylrest bedeuten, $R^1$ und $R^2$ zusammen mit dem benachbarten Kohlenstoffatom oder $R^2$ und $R^3$ zusammen mit den beiden benachbarten Kohlenstoffatomen auch Glieder eines monocyclischen oder bicyclischen, alicyclischen Ringes mit 5 bis 9 Kohlenstoffatomen bezeichnen können, ein oder 2 der Reste $R^1$, $R^2$ und $R^3$ auch jeweils für ein Wasserstoffatom stehen können. Die vorgenannten Reste und Ringe können noch durch unter den Reaktionsbedingungen inerte Gruppen, z.B. Alkylgruppen mit 1 bis 4 Kohlenstoffatomen, substituiert sein.

Beispielsweise sind folgende Alkenylisocyanate als Ausgangsstoffe II geeignet: Propenyl, Butenyl, Pentenyl, Hexenyl, Heptenyl, Octenyl, Nonenyl, Decenyl, Undecenyl, Dodecenyl, 2-Methylpropenyl-, 2-Methylbutenyl-, 2-Methylpentenyl-, 2-Methylhexenyl-, 2-Methylheptenyl-, 2-Methyloctenyl-, 2-Methylnonenyl-, 2-Methyldecenyl-, 2-Methylundecenyl-, 2-Methyldodecenyl-, 2-Äthylbutenyl-, 2-Äthylpentenyl-, 2-Äthylhexenyl-, 2-Äthylheptenyl-, 2-Äthyloctenyl-, 2-Äthylnonenyl-, 2-Äthyldecenyl-, 2-Äthylundecenyl-, 2-Äthyldodecenyl-, 1,2-Dimethylvinyl-, 1,2-Dimethylpropenyl-, 1,2-Dimethylbutenyl-, 1,2-Dimethylpentenyl-, 1,2-Dimethylhexenyl-, 1,2-Dimethylheptenyl-, 1,2-Dimethyloctenyl-, 1,2-Dimethylnonenyl-, 3-Methylbutenyl-, 3-Methylpentenyl-, 3-Methylhexenyl-, 3-Methylheptenyl-, 3-Methyloctenyl-, 3-Methylnonenyl-, 3-Methyldecenyl-, 3-Methylundecenyl-, 3-Methyldodecenyl-, Cyclohexylidenmethyl-, Phenylvinyl-, Benzylvinyl-, (4-Methylphenyl)-vinyl-, Cyclohexylvinyl-, Cyclohex-1-enyl-(1)-, (2'-Norbornyliden)-methyl-, Norbornenyl-(2)-isocyanat.

Die Umsetzung kann bei einer Temperatur von +80°C bis -78°C, wird aber im allgemeinen bei einer Temperatur von +40°C bis -78°C, vorzugsweise von +30 bis -78°C, insbesondere bei 0°C bis -40°C, drucklos oder unter Druck, vorzugsweise bei 0,7 bis 2 bar, kontinuierlich oder diskontinuierlich durchgeführt. Man kann ohne Lösungsmittel umsetzen, zweckmäßig verwendet man aber unter den Reaktionsbedingungen inerte Lösungsmittel. Wasser wird nicht verwendet. Bevorzugt arbeitet man in Lösungsmitteln, die bei der weiteren Umsetzung des Endstoffs, insbesondere der α-Chloralkylcarbamidsäurechloride, als Reaktionsmedien Verwendung finden. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Chlorkohlenwasserstoffe, z.B. Tetrachloräthy-

len, 1,1,2,2- oder 1,1,1,2-Tetrachloräthan, Amylchlorid, Cyclohexylchlorid, 1,2-Dichlorpropan, Methylenchlorid, Dichlorbutan, Isopropylbromid, n-Propylbromid, Butylbromid, Chloroform, Äthyljodid, Propyljodid, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; Äther, z.B. Äthylpropyläther, Methyl-tert.-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thioanisol, ß,ß'-Dichlordiäthyläther; Ketone wie Methyläthylketon, Aceton, Diisopropylketon, Diäthylketon, Methylisobutylketon, Acetophenon, Cyclohexanon, Äthylisoamylketon, Diisobutylketon, Methylcyclohexanon, Dimethylcyclohexanon; Ester wie Methylacetat, n-Propylacetat, Methylpropionat, Butylacetat, Äthylformiat, Phthalsäuremethylester, Benzoesäuremethylester, Essigester, Phenylacetat; aliphatische oder cycloaliphatische Kohlenwasserstoffe, z.B. Pentan, Heptan, Pinan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; und entsprechende Gemische. Zweckmäßig verwendet man das Lösungsmittel in einer Menge von 200 bis 10 000 Gewichtsprozent, vorzugsweise von 300 bis 2 000 Gewichtsprozent, bezogen auf den Ausgangsstoff II.

Die Reaktion kann wie folgt durchgeführt werden: Ein Gemisch der Ausgangsstoffe und zweckmäßig Lösungsmittel wird während 0,1 bis 4 Stunden bei der Reaktionstemperatur gehalten. Ausgangsstoff II wird vorteilhaft im Lösungsmittel vorgelegt und bei der Reaktionstemperatur der Halogenwasserstoff eingeleitet. Im Falle der α-Jodalkylcarbamidsäurejodide ist es vorteilhaft, Jodwasserstoff und Lösungsmittel vorzulegen und dann den Ausgangsstoff II langsam zuzugeben. Zweckmäßig rührt man die Reaktionslösung 0,25 Stunden bis eine Stunde nach. Dann wird der Endstoff aus dem Gemisch in üblicher Weise, z.B. durch Kristallisation und Filtration, abgetrennt.

Die nach dem Verfahren der Erfindung hergestellten α-Halogenalkylcarbamidsäurehalogenide sind wertvolle Ausgangsstoffe für die Herstellung von Lackrohstoffen, Textilbeschichtungsmitteln, Farbstoffen, Pharmaceutica und Pflanzenschutzmitteln. Die neuen Stoffe, vorteilhaft solche der Formel I mit den vorgenannten bevorzugten Resten $R^1$, $R^2$ und $R^3$, insbesondere α-Chlorpropylcarbamidsäurechlorid, α-Chlorbutylcarbamidsäurechlorid, α-Chlorpentylcarbamidsäurechlorid, α-Chlorhexylcarbamidsäurechlorid, α-Chlorheptylcarbamidsäurechlorid, α-Chloroctylcarbamidsäurechlorid, α-Chlornonylcarbamidsäurechlorid, α-Chlordecylcarbamidsäurechlorid, α-Chlorundecylcarbamidsäurechlorid, α-Chlordodecylcarbamidsäurechlorid, α-Brompropylcarbamidsäurebromid, α-Brombutylcarbamidsäurebromid, α-Brompentylcarbamidsäurebromid, α-Bromhexylcarbamidsäurebromid, α-Bromheptylcarbamidsäurebromid, α-Bromoctylcarbamidsäurebromid, α-Bromnonylcarbamidsäurebromid, α-Bromdecylcarbamidsäurebromid, α-Bromundecylcarbamidsäurebromid, α-Bromdodecylcarbamidsäurebromid, 1-Chlor-2-methyl-propylcarbamidsäurechlorid, 1-Brom-1-methyl-propylcarbamidsäurebromid, 1-Chlor-2-methyl-butyl-carbamidsäurechlorid, 1-Brom-2-methyl-butylcarbamidsäurebromid, 1-Chlor-2-methyl-pentylcarbamidsäure-

chlorid, 1-Brom-2-methyl-pentylcarbamidsäurebromid, 1-Chlor-2-methyl-hexylcarbamidsäurechlorid, 1-Brom-2-methyl-hexylcarbamidsäurebromid, 1-Chlor-2-methyl-heptylcarbamidsäurechlorid, 1-Brom-2-methyl-heptylcarbamidsäurebromid, 1-Chlor-2-methyl-octylcarbamidsäurechlorid, 1-Brom-2-methyl-octylcarbamidsäurebromid, 1-Chlor-2-methyl-nonylcarbamidsäurechlorid, 1-Brom-2-methyl-nonylcarbamidsäurebromid, 1-Chlor-2-methyl-decylcarbamidsäurechlorid, 1-Brom-2-methyl-decylcarbamidsäurebromid, 1-Chlor-2-methyl-undecylcarbamidsäurechlorid, 1-Brom-2-methyl-undecylcarbamidsäurebromid, 1-Chlor-1-äthyl-butylcarbamidsäurechlorid, 1-Brom-1-äthylbutylcarbamidsäurebromid, 1-Chlor-2-äthyl-pentylcarbamidsäurechlorid, 1-Brom-2-äthyl-pentylcarbamidsäurebromid, 1-Chlor-2-äthyl-hexylcarbamidsäurechlorid, 1-Brom-2-äthylhexylcarbamidsäurebromid, 1-Chlor-3-methyl-butylcarbamidsäurechlorid, 1-Brom-3-methyl-butylcarbamidsäurebromid, 1-Chlor-3-methyl-pentylcarbamidsäurechlorid, 1-Brom-3-methyl-pentylcarbamidsäurebromid, 1-Chlor-3-methyl-hexylcarbamidsäurechlorid, 1-Brom-3-methyl-hexylcarbamidsäurebromid, 1-Chlor-1-(cyclohexyl)-methylcarbamidsäurechlorid, 1-Brom-1-(cyclohexyl)-methylcarbamidsäurebromid, 1-Chlor-2-phenyl-äthylcarbamidsäurechlorid, 1-Brom-2-phenyl-äthylcarbamidsäurebromid, 1-Chlor-2-phenyl-propylcarbamidsäurechlorid, 1-Brom-2-phenyl-propylcarbamidsäurebromid, Chlor-(2-norbornyl)-methylcarbamidsäurechlorid, Brom-(2-norbornyl)-methylcarbamidsäurebromid, sind für vorgenannte Verwendungen vorteilhafte Stoffe. Die erfindungsgemäßen α-Monohalogenalkylcarbamidsäurechloride sind wertvolle Vorprodukte für α,β-ungesättigte Isocyanate und Carbamidsäurechloride. Diese, wie z.B. das Vinyl- oder Propenyl-isocyanat, sind für weitere industrielle Synthesen interessante Monomere.

Die in den Beispielen genannten Teile sind Gewichtsteile.

Beispiel 1

22 Teile 3-Methylbuten-1-yl-isocyanat werden in 75 Teilen Dichlormethan vorgelegt. In diese Lösung werden bei -39°C innerhalb von 40 Minuten 15 Teile Chlorwasserstoff eingeleitet. Die Reaktionslösung wird weitere 20 Minuten bei dieser Temperatur gerührt. Man erhält nach Filtration 30 Teile (82 % der Theorie) 1-Chlor-3-methyl-butylcarbamidsäurechlorid vom Fp 35°C und NMR-Spektrum in $CDCl_3$ (Standard Tetramethylsilan):

| | |
|---|---|
| $(CH_3-)$ | 0,9 ppm |
| $(-\underset{|}{C}H-CH_2-)$ | 1,7 - 1,9 ppm |
| $(Cl-\underset{|}{\overset{|}{C}}-H)$ | 5,6 ppm |
| (NH) | 6,5 ppm |

Beispiel 2

21 Teile Cyclohexylidenmethylisocyanat werden in 80 Teilen Dichlormethan vorgelegt. In diese Lösung werden bei -39°C innerhalb 30 Minuten 8 Teile Chlorwasserstoff eingeleitet. Die Reaktionslösung wird weitere 45 Minuten bei dieser Temperatur gerührt. Nach Abziehen des Lösungsmittels bei -20°C erhält man 31 Teile (96 % der Theorie) des flüssigen Chlor-(cyclohexyl)-methylcarbamidsäurechlorids. NMR-Spektrum in $CDCl_3$ (Standard Tetramethylsilan):

| | |
|---|---|
| $(C_6H_{11}-CH_2-)$ | 1-2 ppm |
| $(Cl-\underset{|}{\overset{|}{C}}-H$ | 5,5 ppm |
| (NH) | 6,5 ppm |

Beispiel 3

80 Teile Buten-1-yl-isocyanat werden in 400 Teilen Chloroform vorgelegt. Innerhalb von 1,5 Stunden werden bei -30°C in die Lösung 62 Teile Chlorwasserstoff eingeleitet. Die Reaktionslösung wird weitere 30 minuten bei -20°C gerührt. Man erhält nach Filtration 128 Teile (91 % der Theorie) α-Chlorbutylcarbamidsäurechlorid vom Fp 5 bis 10°C und NMR-Spektrum in $CDCl_3$ (Standard Tetramethylsilan):

| | |
|---|---|
| ($-CH_3$) | 0,9 ppm |
| ($-CH_2-$) | 1,4 - 1,6 ppm |
| (Cl-CH) | 5,7 ppm |
| (NH) | 6,6 ppm |

Beispiel 4

54 Teile Propen-1-yl-isocyanat werden in 200 Teilen Tetrachlorkohlenstoff vorgelegt. Bei -20°C werden in diese Lösung 105 Teile Bromwasserstoff innerhalb von 50 Minuten eingeleitet. Die Reaktionslösung wird weitere 60 Minuten bei -20°C gerührt. Man erhält nach Filtration 139 Teile (86 % der Theorie) des flüssigen α-Brompropylcarbamidsäurebromids. NMR-Spektrum in $CDCl_3$ (Standard Tetramethylsilan):

| | |
|---|---|
| ($CH_3$) | 1,0 ppm |
| ($-CH_2-$) | 1,7 ppm |
| (Br-C-H) | 5,8 ppm |
| (NH) | 6,5 ppm |

Beispiel 5

80 Teile Jodwasserstoff werden in 130 Teilen Chloroform bei -50°C vorgelegt. Innerhalb von 40 Minuten werden zu dieser Lösung 25 Teile 1-Propenylisocyanat langsam zugegeben. Die Reaktionslösung wird weitere 20 Minuten bei -50°C gerührt. Man erhält nach Filtration 78 Teile (77 % der Theorie) α-Jodpropylcarbamidsäurejodid mit einem Zersetzungspunkt bei 10°C und NMR-Spektrum in $CDCl_3$ (Standard Tetramethylsilan):

| | |
|---|---|
| ($-CH_3$) | 1,0 ppm |
| ($-CH_2-$) | 2,1 ppm |
| (J-CH) | 5,9 ppm |
| (NH) | 7,1 ppm |

Beispiele 6 bis 10

Analog zu Beispiel 4 (Menge und Molverhältnis von Ausgangsstoff II sowie Bedingungen von Reaktion und Aufarbeitung) werden folgende Endstoffe I hergestellt:

| Beispiel | | $^1$H-NMR-Spektrum ($CDCl_3$) $\delta$ (ppm) | Ausbeute in % der Theorie |
|---|---|---|---|
| 6 | 1-Chlorpropylcarbamidsäurechlorid | 1,1 (3)t<br>2,0 (2)p<br>5,6 (1)d,t<br>6,8 (1)breit | 97 |
| 7 | 1-Brombutylcarbamidsäurebromid | 0,9 (3)t<br>1,4 (2)m<br>1,9 (2)m<br>5,7 (1)m<br>6,5 (1)breit | 95 |
| 8 | 1-Brom-2-methylbutylcarbamidsäure-bromid | 1,0 (6)t<br>1,4-2,0 (3)m<br>5,9 (1)d,d<br>6,5 (1)breit | 86 |
| 9 | 1-Bromoctylcarbamidsäurebromid | 0,9 (3)t<br>1,3 (10)s,breit<br>2,1 (2)m<br>5,8 (1)m<br>6,9 (1)breit | 94 |
| 10 | 1-Brom-2-phenylpropylcarbamidsäure-bromid | 2,0 (3)s<br>3,3 (1)m<br>6,2 (1)m<br>6,85 (1)breit<br>7,3 (5)s | 88 |

Beispiel 11

Analog zu Beispiel 5 (Menge und Molverhältnis von Ausgangsstoff II sowie Bedingungen von Reaktion und Aufarbeitung) wird folgender Endstoff I hergestellt:

| | $^1$H-NMR-Spektrum ($CDCl_3$) $\delta$ (ppm) | Ausbeute in % der Theorie |
|---|---|---|
| 1-Jodbutylcarbamidsäurejodid | 1,0 (3)t<br>1,5 (2)m<br>2,0 (2)m<br>6,0 (1)m<br>6,5 (1)breit | 83 |

## Patentansprüche

1. Verfahren zur Herstellung von α-Halogenalkylcarbamidsäurehalogeniden der Formel

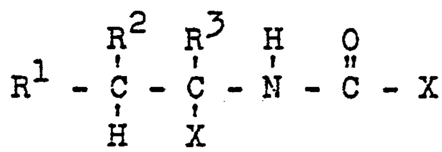

I,

worin die einzelnen Reste $R^1$, $R^2$ und $R^3$ gleich oder verschieden sein können und jeweils einen aliphatischen, cycloaliphatischen, araliphatischen, aliphatisch-aromatischen oder aromatischen Rest bedeuten, $R^1$ und $R^2$ zusammen mit dem benachbarten Kohlenstoffatom oder $R^2$ und $R^3$ zusammen mit den beiden benachbarten Kohlenstoffatomen auch Glieder eines alicyclischen Ringes bezeichnen können, ein oder zwei der Reste $R^1$, $R^2$ und $R^3$ auch jeweils für ein Wasserstoffatom stehen können, X ein Halogenatom bedeutet, dadurch gekennzeichnet, daß man Alkenylisocyanate der Formel

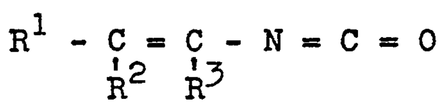

II,

worin $R^1$, $R^2$ und $R^3$ die vorgenannte Bedeutung besitzen, mit Halogenwasserstoff bei einer Temperatur von -78°C bis +80°C umsetzt.

2. α-Halogenalkylcarbamidsäurehalogenide der Formel

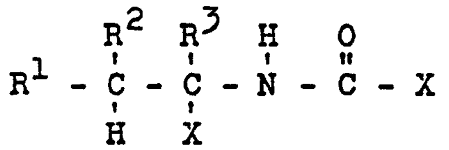

I,

worin die einzelnen Reste $R^1$, $R^2$ und $R^3$ gleich oder verschieden sein können und jeweils einen Alkylrest mit 1 bis 12 Kohlenstoffatomen, einen Cycloalkylrest mit 5 bis 6 Kohlenstoffatomen, einen Aralkylrest oder Alkylarylrest mit 7 bis 12 Kohlenstoffatomen, einen Phenylrest bedeuten, $R^1$ und $R^2$ zusammen mit dem benachbarten Kohlenstoffatom oder $R^2$ und $R^3$ zusammen mit den benachbarten Kohlenstoffatomen auch Glieder eines alicyclischen Ringes bezeichnen können, ein oder zwei der Reste $R^1$, $R^2$ und $R^3$ auch jeweils für ein Wasserstoffatom stehen können, X ein Halogenatom bedeutet, wobei, wenn $R^2$ und $R^3$ gleichzeitig ein Wasserstoffatom bedeuten, $R^1$ für einen cycloaliphatischen, araliphatischen, aliphatisch-aromatischen oder aromatischen Rest steht.

Europäisches Patentamt

# EUROPÄISCHER RECHERCHENBERICHT

Nummer der Anmeldung

EP 79 102 313.8

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| P,X | EP - A1 - 0 000 362 (BASF)<br>* Ansprüche 1 und 2 *<br>-- | 1,2 | C 07 C 125/03<br>C 07 C 125/00 |
| D,A | ANGEWANDTE CHEMIE, 74. Jahrgang, Nr. 13, 1962<br>Weinheim<br>H. HOLTSCHMIDT "Hochtemperatur-Chlorierung von Aminen und Acylaminen"<br>Seiten 848 bis 855<br>-- | | |
| A | DE - A - 2 146 069 (BAYER)<br>---- | | |

RECHERCHIERTE SACHGEBIETE (Int. Cl.3)

C 07 C 125/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 14-11-1979 | STOOS |

EPA form 1503.1 06.78